# EUROPEAN PATENT APPLICATION

(11) **EP 1 457 570 A1**
(43) Date of publication of application: **15.09.2004**
(21) Application number: 02788671.2
(22) Date of filing: 28.11.2002
(51) Int. Cl.: C12P 41/00, C07C 213/02, C07C 217/74, C07C 309/66, C07C 309/73, C07B 55/00

(54) **PROCESS FOR PREPARATION OF 2-AMINOTETRALIN DERIVATIVES AND INTERMEDIATES THEREOF**

(30) Priority: 28.11.2001 JP 2001363336; 28.08.2002 JP 2002248888
(71) Applicant: KANEKA CORPORATION, Osaka-shi, Osaka 530-8288 (JP)
(72) Inventor: HONDA, Tatsuya, c/o KANEKA CORPORATION, Takasago-shi, Hyogo 678-8688 (JP); FUJII, Akio, c/o KANEKA CORPORATION, Takasago-shi, Hyogo 676-8688 (JP); INOUE, Kenji, c/o KANEKA CORPORATION, Takasago-shi, Hyogo 676-8688 (JP); YASOHARA, Yoshihiko, c/o KANEKA CORPORATION, Takasago-shi, Hyogo 676-8688 (JP); ITAGAKI, Yoshifumi, c/o KANEKA CORPORATION, Takasago-shi, Hyogo 676-8688 (JP); MAEHARA, Katsuji, c/o KANEKA CORPORATION, Takasago-shi, Hyogo 676-8688 (JP); TAKEDA, Toshihiro, c/o KANEKA CORPORATION, Takasago-shi, Hyogo 676-8688 (JP); UEDA, Yasuyoshi, c/o KANEKA CORPORATION, Takasago-shi, Hyogo 676-8688 (JP)
(74) Representative: HOFFMANN - EITLE
(86) International application number: PCT/JP2002/012409
(87) International publication number: WO 2003/046197

(57) **Abstract**

The present invention is to efficiently and simply prepare an optically active 7-substituted-2-aminotetralin with industrial advantage.

In the process, a 7-substituted-2-tetralone or its bisulfite adduct is reduced with a microorganism to an optically active 7-substituted-2-tetralol. Then, a sulfonyl group is introduced to the hydroxy group to form an optically active 7-substituted-2-sulfonyloxytetralin. Then, with inversion of the configuration, a nitrogen substituent is introduced using a nitrogen nucleophile to form an optically active 2,7-substituted tetralin. Furthermore, if necessary, the nitrogen substituent is converted into a non-substituted amino group. Thus, an optically active 7-substituted-2-aminotetralin or its salt is prepared.

## Description

### Technical Field

The present invention relates to processes for preparation of 2-aminotetralin derivatives and their intermediates significantly useful as synthesized intermediates of medicine, and to important intermediates in the process. More specifically, the present invention relates to processes for preparing 7-substituted-2-tetralol by enzymically reducing 7-substituted-2-tetralone and for deriving 7-substituted-2-aminotetralin from 7-substituted-2-tetralol, and to intermediates in the processes.

### Background Art

Optically active 2-tetralol derivatives and 2-aminotetralin derivatives have been conventionally prepared by the following processes:
(Processes for preparing optically active 2-tetralol derivatives)
(1) The process of allowing bakers' yeast (Tetrahedron, Vol. 51, pp. 11,531-11,546, 1995), a microorganism of the genus *Sporobolomyces* (Journal of Chemical Society, Parkin Transactions 1, pp. 3141-3144, 1992), or a microorganism of the genus *Trichosporon* (Journal of Fermentation and Bioengineering, Vol. 81, pp. 304-309, 1996) to act on a 2-tetralone derivative.
   (Processes for preparing optically active 2-aminotetralin derivatives)
(2) The process (Japanese Unexamined Patent Application Publication Nos. 11-228511 and 2000-7624) in which (S)-N-methoxycarbonyl-p-methoxyhomophenylalanine acid chloride is cyclized to (S)-N-methoxycarbonyl-7-methoxy-2-aminotetralin-1-one in the presence of titanium tetrachloride; the ketone is reduced with sodium borohydride to (S)-N-methoxycarbonyl-7-methoxy-1-hydroxy-2-aminotetralin, followed by allowing sodium hydride to act to form an optically active oxazolidinone derivative; and finally the oxazolidinone derivative is subjected to hydrogenolysis to yield (S)-7-methoxy-2-aminotetralin.
(3) The process in which a Schiff base synthesized from 7-methoxy-2-tetralone and (R)-phenethylamine is reduced with sodium borohydride to (S)-N-phenethyl-7-methoxy-2-aminotetralin; the product is subjected to hydrogenolysis to (S)-7-methoxy-2-aminotetralin, followed by forming a diastereomeric salt with mandelic acid; and then the salt is recrystallized to increase the optical purity (Japanese Unexamined Patent Application Publication No. 10-72411).
(4) The process in which 6-bromo-2-tetralone is reduced with a microorganism to (S)-6-bromo-2-tetralol; the hydroxyl group is mesylated with mesyl chloride and replaced with sodium azide to form an azide; then the azide is reduced with sodium borohydride and cobalt bromide to (R)-6-bromo-2-aminotetralin (Journal of Organic Chemistry, Vol. 60, p. 4324, 1995).

However, in process (1), the optical purity of the resulting compound is low and the microorganism has low capability of transforming the substrate. Therefore, this process does not necessarily lead to satisfactory results. Process (2) has relatively large number of steps, and requires another several steps to synthesize the starting material (S)-N-methoxycarbonyl-p-methoxyhomophenylalanine. In addition, the process needs to use titanium tetrachloride or chlorobenzene, which require careful handling, in the step of Friedel-Crafts cyclization and sodium hydride, which also require careful handling, in the step of forming oxazolidinone. In process (3), the resulting targeted compound has a low optical purity. Accordingly, the optical purity must be increased by separation with mandelic acid. Thus, the process is economically inefficient. Process (4) is simple and thus relatively favorable. However, the process uses expensive sodium borohydride requiring careful handling. In the step of reducing the azido group to an amino group, applying a simple hydrogenolysis causes the bromine atom substituted on the benzene ring to be hydrogenated undesirably. The present invention does not need to take into account these disadvantages.

Thus, any process in the known art has problems to be overcome. In view of the above-described circumstances, the object of the present invention is to provide an efficient, economical, industrially advantageous process for preparing 2-aminotetralin derivatives and to provide important intermediates of the 2-aminotetralin derivatives.

### Summary of Invention

The inventors have conducted research to overcome the problems by various approaches and, consequently accomplished the present invention.

Specifically, the present invention relates to a process for preparing an optically active 7-substituted-2-tetralol (2). In the process, a 7-substituted-2-tetralone or its bisulfite adduct is reduced with a culture broth of microorganism, cell, or a material derived therefrom capable of transforming the 7-substituted-2-tetralone or its bisulfite adduct into the optically active 7-substituted-2-tetralol, wherein the microorganism is a microorganism belonging to a genus selected from the group consisting of *Candida, Debaryomyces, Pichia, Kluyveromyces, Metschnikowia,* *Ogataea, Sporidiobolus, Torulaspora, Geotrichum, Yamadazyma, Endomyces, Dipodascus, Saccharomycopsis, Issatchenkia, Kuraishia, Lipomyces, Lodderomyces, Rhodosporidium, Rhodotorula, Sporobolomyces, Saturnispora, Zygosaccharomyces, Cellulomonas, Jensenia, Arthrobacter, Acidiphilium, Pseudomonas, Rhodococcus, Devosia,* and *Micrococcus.* The 7-substituted-2-tetralone is expressed by general formula (1): 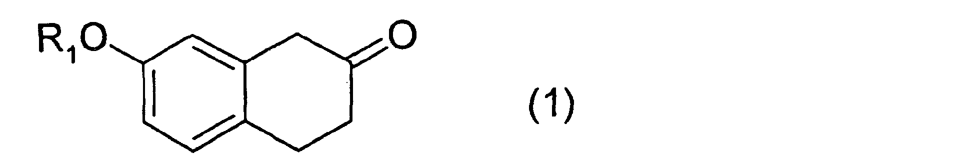 (wherein R₁ represents hydrogen, an alkyl group having 1 to 10 carbon atoms, an aryl group having 6 to 20 carbon atoms, or an aralkyl group having 7 to 20 carbon atoms). The optically active 7-substituted-2-tetralol is expressed by general formula (2): 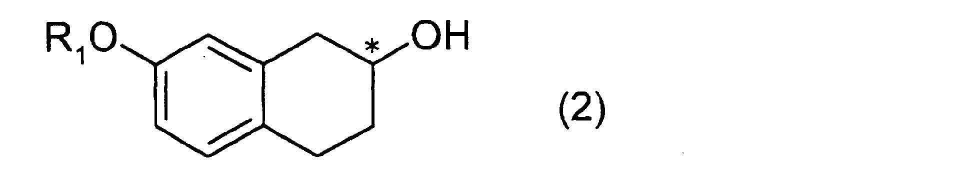 (wherein R₁ represents the same as above, and * represents an asymmetric carbon atom).

The present invention also relates to a process for preparing an optically active 7-substituted-2-aminotetralin or a salt thereof. In the process, a sulfonyl group is introduced to the hydroxy group of an optically active 7-substituted-2-tetralol to form an optically active 7-substituted-2-sulfonyloxytetralin. The optically active 7-substituted-2-tetralol is expressed by general formula (2): 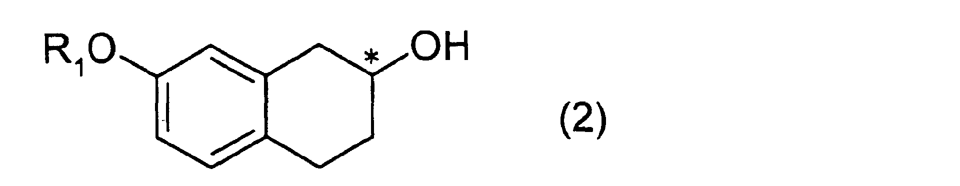 (wherein * represents an asymmetric carbon atom and R₁ represents hydrogen, an alkyl group having 1 to 10 carbon atoms, an aryl group having 6 to 20 carbon atoms, or an aralkyl group having 7 to 20 carbon atoms). The optically active 7-substituted-2-sulfonyloxytetralin is expressed by general formula (3): 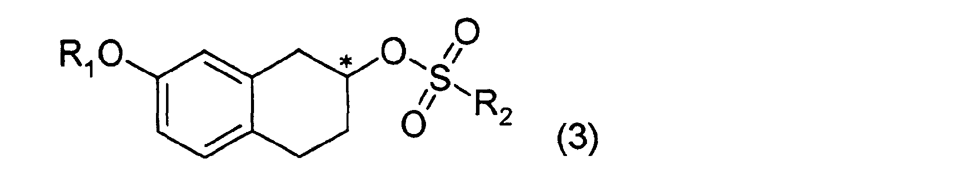 (wherein * and R₁ represent the same as above, and R₂ represents an alkyl group having 1 to 10 carbon atoms, an aryl group having 6 to 20 carbon atoms, an aralkyl group having 7 to 20 carbon atoms, a substituted amino group, or a hydroxy group). Then, a nitrogen substituent is introduced using a nitrogen nucleophile to form an optically active 2,7-substituted tetralin with inversion of the configuration. The optically active 2,7-substituted tetralin is expressed by general formula (4): 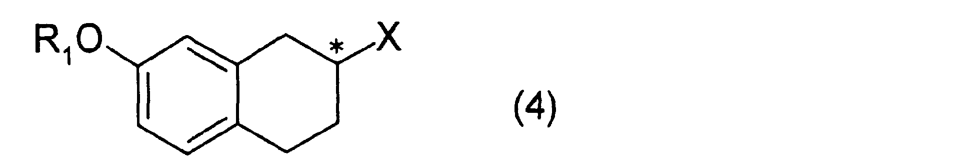 (wherein * and R₁ represents the same as above, and X represents a non-substituted amino group, an alkylamino group having 1 to 10 carbon atoms, an arylamino group having 6 to 20 carbon atoms, an aralkylamino group having 7 to 20 carbon atoms, an amido group having 1 to 20 carbon atoms, an imido group having 2 to 20 carbon atoms, a sulfonylamino group having 1 to 20 carbon atoms, or an azido group). Furthermore, if necessary, the nitrogen substituent is transformed to a non-substituted amino group. The resulting optically active 7-substituted-2-aminotetralin is expressed by general formula (5): 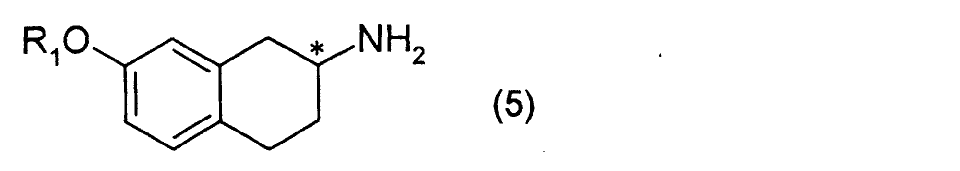 (wherein * and R₁ represents the same as above).

The present invention also relates to an optically active 7-substituted-2-sulfonyloxytetralin expressed by general formula (3): 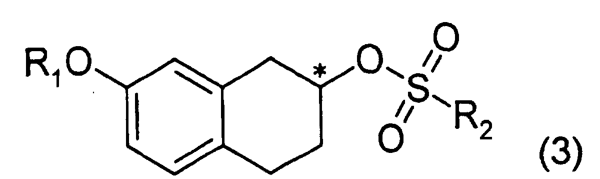 (wherein * represents an asymmetric carbon atom, and R₁ represents hydrogen, an alkyl group having 1 to 10 carbon atoms, an aryl group having 6 to 20 carbon atoms, or an aralkyl group having 7 to 20 carbon atoms, and R₂ represents an alkyl group having 1 to 10 carbon atoms, an aryl group having 6 to 20 carbon atoms, an aralkyl group having 7 to 20 carbon atoms, a substituted amino group, or a hydroxy group).

### Disclosure of Invention

The present invention will be further described in detail.

First, a process for converting a 7-substituted-2-tetralone (1) into an optically active 7-substituted-2-tetralol (2) by microbial reduction will be described.

In the 7-substituted-2-tetralone (1) used in the present invention, R₁ represents hydrogen, an alkyl group having 1 to 10 carbon atoms, an aryl group having 6 to 20 carbon atoms, or an aralkyl group having 7 to 20 carbon atoms.

The alkyl group having 1 to 10 carbon atoms, the aryl group having 6 to 20 carbon atoms, and the aralkyl group having 7 to 20 carbon atoms may have a substituent, such as a halogen, an alkyl group having 1 to 10 carbon atoms, or a nitro group.

For example, alkyl groups having 1 to 10 carbon atoms include methyl, ethyl, n-propyl, isopropyl, n-butyl, isobutyl, tert-butyl, and trifluoromethyl. Among these, the methyl group is preferable because it can be deprotected in a downstream step. For example, aryl groups having 6 to 20 carbon atoms include phenyl, p-methylphenyl, o-nitrophenyl, m-nitrophenyl, p-nitrophenyl, 2,4-dinitrophenyl, and p-chlorophenyl. The aralkyl group having 7 to 20 carbon atoms is, for example, a benzyl group.

Particularly preferably, R₁ is the methyl group.

The description of R₁ is applied to general formulas (2) to (5).

In the description herein, * represents an asymmetric carbon atom.

If the 7-substituted-2-tetralone (1) is, for example, 7-methoxy-2-tetralone, it can be synthesized by reducing easily available 2,7-dimethoxynaphthalene by Birch reduction (Tetrahedron Letters, Vol. 31, p. 875 (1990)).

In the present invention, a bisulfite adduct of the 7-substituted-2-tetralone (1) may be used in the same manner. The bisulfite adduct can be prepared by treating the 7-substituted-2-tetralone (1) with a bisulfite. Since the bisulfite adduct is of crystalline solid, it is superior in stability and workability to carbonyl compounds and is often easy to use accordingly.

For example, bisulfites used in the treatment above include potassium bisulfite, sodium bisulfite, and calcium bisulfite. Preferably, sodium bisulfite is used.

The treatment is carried out by using water or a mixed solvent containing water and an organic solvent miscible with water and nonreactive with the bisulfite. A solution of the bisulfite may be added to a solution of the 7-substituted-2-tetralone (1), or the solution of the 7-substituted-2-tetralone (1) may be added to the bisulfite solution. Exemplary solvents miscible with water and nonreactive with the bisulfite include, but not limited to, 1,2-dimethoxyethane, 1,4-dioxane, tetrahydrofuran, diethylene glycol dimethyl ether, triethylene glycol dimethyl ether, polyethylene glycol dimethyl ether, acetonitrile, methanol, ethanol, n-propanol, isopropanol, and t-butanol.

The bisulfite is used in an amount in the range of, for example, 1 to 100 equivalents relative to the 7-substituted-2-tetralone (1) normally, but the amount depends on the types of metal salt and solvent used and treating conditions. Preferably, the lower limit is 1 equivalent and the upper limit is 20 equivalents, and more preferably the lower limit is 1 equivalent and the upper limit is 10 equivalents, from the viewpoint of economical efficiency.

The microorganism for converting the 7-substituted-2-tetralone (1) into the optically active 7-substituted-2-tetralol (2) may be selected by, for example, the following process.

First, microbial cells are collected from 5 mL of a culture broth of a microorganism by centrifugation or the like and suspended in 1 mL of a 100 mM phosphate buffer solution (pH 6.5) containing 5 mg of 7-methoxy-2-tetralone and 5 mg of glucose. The suspension is shaken in a test tube at 30°C for 2 to 3 days. For evaluation of the reduction capability, for example, the reaction mixture after the shaking reaction is subjected to extraction with ethyl acetate, and optically active 7-methoxy-2-tetralol produced in the extract is analyzed by high-performance liquid chromatography. If the optically active 7-methoxy-2-tetralol is produced, the microorganism is accepted.

The microorganism used in the present invention is selected on the basis of satisfactory results of the above-described test from among bacteria, actinomycete, mold, yeast, and fungi imperfecti. In particular, a microorganism is preferably used which belongs to a genus selected from the group consisting of *Candida, Debaryomyces, Pichia, Kluyveromyces, Metschnikowia, Ogataea, Sporidiobolus,* *Torulaspora, Geotrichum, Yamadazyma, Endomyces, Dipodascus, Saccharomycopsis, Issatchenkia, Kuraishia, Lipomyces, Lodderomyces, Rhodosporidium, Rhodotorula, Sporobolomyces, Saturnispora, Zygosaccharomyces, Cellulomonas, Jensenia, Arthrobacter, Acidiphilium, Pseudomonas, Rhodococcus, Devosia,* and *Micrococcus.* Among these genera, Candida, *Sporidiobolus, Yamadazyma, Acidiphilium, Pseudomonas,* and Devosia are more preferable.

For a 7-substituted-2-tetralol in an (R) form, preferred microorganisms belong to a genus selected from the group consisting of *Candida, Debaryomyces, Pichia, Kluyveromyces, Metschnikowia, Ogataea, Sporidiobolus, Torulaspora, Geotrichum, Yamadazyma, Arthrobacter, Acidiphilium, Pseudomonas, Rhodococcus,* and *Devosia.* Among these genera, *Candida, Sporidiobolus, Yamadazyma, Acidiphilium, Pseudomonas,* and *Devosia* are more preferable. Specifically, for example, microorganisms include *Candida magnoliae* IFO705, *Candida maris* IFO10003, *Candida catenulate* IFO0745, *Candida glabrata* IFO0005, *Candida maltosa* IFO1976, Candida *albicans* IFO1594, *Candida fennica* CBS6087, *Debaryomyces hansenii var. hansenii* IFO0019, *Pichia* anomala IF00118, *Kluyveromyces polysporus* IFO0996, *Metschnokowia bicuspidata var. bicuspidata* IFO1408, *Ogataeaminuta var. nonfermentans* IFO1473, *Sporidiobolus johnsonii* IFO6903, *Tolulaspora delbrueckii* IFO0381, *Geotrichum fermentans* IFO1199, *Yamadazyma farinosa* IFO0534, *Arthrobacter protophormiae* IFO12128, *Acidiphilium cryptum* IFO14242, *Pseudomonas putida* IFO14164, *Rhodococcus erythropolis* IFO12320, and *Devosia riboflavina* IFO13584.

For a 7-substituted-2-tetralol in an (S) form, preferred microorganisms belong to a genus selected from the group consisting of *Candida, Debaryomyces, Endomyces, Dipodascus, Saccharomycopsis, Issatchenkia, Kuraishia, Lipomyces, Lodderomyces, Pichia, Rhodosporidium, Rhodotorula, Sporobolomyces, Sporidiobolus, Saturnispora, Zygosaccharomyces, Cellulomonas, Jensenia, Micrococcus, Rhodococcus,* and *Metschnikowia.* Among these genera, *Candida*, *Debaryomyces, Dipodascus,* and *Rhodococcus* are more preferable. Specifically, exemplary microorganisms include *Candida glaebosa* IFO1353, *Candida haemulonii* IFO10001, *Candida holmii* IFO0660, *Candida intermedia* IF00761, *Candida boidinii* IFO10240, *Candida pintolopesii* IFO0729, *Candida oleophila* IFO1021, *Candida sonorensis* IFO10027, *Candida tropicalis* IF00618, *Debaryomyces carsonii* IFO0946, *Endomyces decipiens* IF00102, *Dipodascus ovetensis* IFO1201, *Saccharomycopsis selenospora* IFO1850, *Issatchenkia terricola* IF00933, *Kuraishia capsulate* IF00721, *Lipomyces starkeyi* IFO0678, *Lodderomyces elongisporus* IFO1676, *Metschnikowia gruessii* IFO0749, *Pichia wickerhamii* IFO1278, *Rhodosporidium toruloides* IFO0559, *Rhodotorula araucariae* IFO10053, *Sporobolomyces salmonicolor* IFO1038, *Sporidiobolus holsaticus* IF01032, *Debaryomyces occidentalis var. occidentalis* IF00371, *Saturnispora dispora* IFO0035, Candida *stellata* IF00703, *Zygosaccharomyces bailii* IF00519, *Zygosaccharomyces bailii* IFO0488, *Zygosaccharomyces bailii* IFO0493, *Cellulomonas fimi* IFO15513, *Jensenia canicruria* IFO13914, *Micrococcus luteus* IFO13867, and *Rhodococcus erythropolis* IAM1474.

These microorganisms can be obtained from stock strains readily available or purchasable or can be isolated from the natural world. It is also possible to obtain strains having favorable properties for the reaction by causing mutation of these microorganisms.

In culturing these microorganisms, any of the media containing nutrient sources assimilable by these microorganisms can generally be used. For example, used are ordinary media prepared by mixing and incorporating carbon sources, for example, saccharide, such as glucose, sucrose, and maltose, organic acids such as lactic acid, acetic acid, citric acid, and propionic acid, alcohols such as ethanol and glycerin, hydrocarbons such as paraffins, fats and oils such as soybean oil and rapeseed oil, or mixtures of these; nitrogen sources such as ammonium sulfate, ammonium phosphate, urea, yeast extract, meat extract, peptone, and corn steep liquor, and, further, other nutrient sources, for example, other inorganic salts and vitamins.

The culture broth of the microorganism can be generally carried out under ordinary conditions, preferably at a pH of 4.0 to 9.5 and a temperature within the range of 20°C to 45°C, more preferably at a pH of 5 to 8 and a temperature within the range of 25 to 40°C, under aerobic conditions for 10 to 96 hours, for instance.

In the reaction of the microorganism with the 7-substituted-2-tetralone (1), the culture broth containing cells of the above microorganisms can be generally used as it is. The culture broth may also be used in a concentrated form. In cases a certain component in the culture broth adversely affect the reaction, preferably, microbial cells or a material derived therefrom obtained by centrifugation of the culture broth may also be used.

The material derived from cells of the microorganism is not particularly limited, but includes dried cells obtained by dehydration treatment with acetone or diphosphorus pentaoxide or by drying using a desiccator or electric fan, materials derived by surfactant treatment, materials derived by lytic enzyme treatment, immobilized cells, and cell-free extract preparations derived from disruption of cells. It is also possible to use an enzyme catalyzing enantioselective reduction reaction, purified from a culture broth.

In the reduction reaction, the substrate 7-substituted-2-tetralone or its bisulfite adduct may be added all at once in an early stage of the reaction or in divided portions with the progress of the reaction.

The temperature during the reaction is generally 10 to 60°C, and preferably 20 to 40°C, and the pH during the reaction is within the range of 2.5 to 9, preferably 5 to 9.

The content of the culture broth of microorganism, cell, or a material derived therefrom (hereinafter referred to as the culture broth or the like of the microorganism) in the reaction mixture can be appropriately selected according to the ability thereof to reduce the substrate.

The concentration of the substrate in the reaction mixture is preferably 0.01% to 50% (w/v), more preferably 0.1% to 30% (w/v).

The above reaction is generally carried out with shaking or with aeration and stirring. The reaction time is appropriately selected according to the concentrations of the substrate and the culture broth or the like of the microorganism and other reaction conditions. In general, it is preferable to select such reaction conditions that the reaction is completed in 2 to 168 hours.

For promoting the reduction reaction, the addition, in an amount of 1% to 30% (w/v), of an energy source, such as glucose or ethanol, is preferable to obtain better results.

The reaction can also be promoted by adding a coenzyme generally required for biological reduction reaction, and such coenzymes include reduced nicotinamide adenine dinucleotide (NADH) and reduced nicotinamide adenine dinucleotide phosphate (NADPH). Specifically, the coenzyme may be directly added to the reaction mixture, or a reaction system producing NADH or NADPH may be added together with an oxidized coenzyme. For example, such reaction systems include those which reduce NAD to NADH when formic acid dehydrogenase produces carbon dioxide and water from formic acid, and those which reduce NAD to NADH or NADP to NADPH when glucose dehydrogenase produces gluconolactone from glucose.

It is also effective to add a surfactant, such as Triton (produced by Nacalai Tesque), Span (produced by Kanto Kagaku), or Tween (produced by Nacalai Tesque), to the reaction mixture.

In order to prevent the substrate and/or alcohol produced by the reduction reaction from inhibiting the reaction, a water-insoluble organic solvent, such as ethyl acetate, butyl acetate, isopropyl ether, or toluene, may also be added to the reaction mixture. Furthermore, in order to increase the solubility of the substrate, a water-soluble organic solvent may be added, such as methanol, ethanol, acetone, tetrahydrofuran, or dimethylsulfoxide.

The optically active 7-substituted-2-tetralol (2) produced by the reduction may be extracted with a solvent, such as ethyl acetate or toluene, directly or after separation of cells and so on, and the solvent is removed to collect the compound. Additional purification by, for example, silica gel column chromatography or recrystallization can increase the purity of the compound.

In the subsequent step, a sulfonyl group is introduced to the hydroxy group of the optically active 7-substituted-2-tetralol (2) to form an optically active 7-substituted-2-sulfonyloxytetralin (3).

The optically active 7-substituted-2-sulfonyloxytetralin expressed by general formula (3) is a new compound and it will be advantageously used for preparing 2-aminotetralin derivatives, which are useful as a synthesized intermediate of medicine.

R₂ represents an alkyl group having 1 to 10 carbon atoms, an aryl group having 6 to 20 carbon atoms, an aralkyl group having 7 to 20 carbon atoms, a substituted amino group, or a hydroxy group. The alkyl group having 1 to 10 carbon atoms, the aryl group having 6 to 20 carbon atoms, and the aralkyl group having 7 to 20 carbon atoms may have a substituent, such as a halogen, an alkyl group having 1 to 10 carbon atoms, or a nitro group.

Exemplary alkyl groups having 1 to 10 carbon atoms include methyl, ethyl, and trifluoromethyl. Exemplary aryl groups having 6 to 20 carbon atoms include phenyl, p-methylphenyl, o-nitrophenyl, m-nitrophenyl, p-nitrophenyl, 2,4-dinitrophenyl, and p-chlorophenyl. The aralkyl group having 7 to 20 carbon atoms is, for example, a benzyl group. The substituted amino group is, for example, dimethylamino group.

Preferably, R₂ is methyl, phenyl, p-methylphenyl, o-nitrophenyl, m-nitrophenyl, or p-nitrophenyl from the viewpoint of increasing the yield of the subsequent substitution reaction.

For introducing a sulfonyl group to the optically active 7-substituted-2-tetralol (2), a sulfonylating agent is used, such as methanesulfonyl chloride, trifluoromethanesulfonyl chloride, benzenesulfonyl chloride, p-toluenesulfonyl chloride, o-nitrobenzenesulfonyl chloride, m-nitrobenzenesulfonyl chloride, p-nitrobenzenesulfonyl chloride, 2,4-dinitrobenzenesulfonyl chloride, p-chlorobenzenesulfonyl chloride, and dimethylaminosulfonyl chloride. Preferably, methanesulfonyl chloride, benzenesulfonyl chloride, or p-toluenesulfonyl chloride is used from the viewpoint of ease of handling and inexpensive availability.

The amount of sulfonylating agent is not particularly limited, but, normally, 1 mole equivalent or more of sulfonylating agent is used relative to the optically active 7-substituted-2-tetralol (2). The preferred amount is generally 10.0 mole equivalents or less, more preferably 5.0 mole equivalents or less, and still more preferably 2.0 mole equivalents or less, from the viewpoint of economical efficiency.

Since typical sulfonylation generates an acid in an amount equivalent to that of the substrate in a mole basis, a base is added. Exemplary bases include: organic bases, such as triethylamine, pyridine, 4-dimethylaminopyridine, and diisopropylethylamine; and inorganic bases, such as sodium hydroxide, sodium carbonate, and sodium hydrogencarbonate. Among these bases, triethylamine and pyridine are preferable from the viewpoint of yield and economical efficiency.

The amount of the base is not particularly limited, but, normally, 1 mole equivalent or more of base is used relative to the optically active 7-substituted-2-tetralol (2). The preferred amount is generally 10.0 mole equivalents or less, more preferably 5.0 mole equivalents or less, and still more preferably 2.0 mole equivalents or less, from the viewpoint of economical efficiency. In particular, pyridine, which is volatile, can be used as a solvent.

The reaction solvent used for the sulfonylation is not particularly limited as long as it does not inhibit the reaction. Exemplary solvents include: hydrocarbon solvents, such as pentane, hexane, heptane, cyclohexane, and petroleum ether; esters, such as ethyl acetate, methyl acetate, propyl acetate, and methyl propionate; aromatic hydrocarbons, such as toluene, benzene, and xylene; nitriles, such as acetonitrile and propionitrile; ethers, such as tert-butyl methyl ether, diethyl ether, diisopropyl ether, tetrahydrofuran, and dioxane; ketones, such as acetone and ethyl methyl ketone; amides, such as N,N-dimethylformamide and N,N-dimethylacetamide; sulfoxides, such as dimethylsulfoxide; halogenated hydrocarbons, such as methylene chloride, 1,2-dichloroethylene, chloroform, and carbon tetrachloride; and amines, such as pyridine and triethylamine. These solvents may be used singly or in combination. Among these, preferred solvents are toluene, ethyl acetate, acetonitrile, dioxane, tetrahydrofuran, methylene chloride, 1,2-dichloroethylene, and their mixture from the viewpoint of the solubility of the optically active 7-substituted-2-tetralol (2) and the stability against the sulfonylating agent. In use of a mixed solvent, the mixing ratio is not particularly limited.

The concentration of the optically active 7-substituted-2-tetralol (2) in the sulfonylation depends on the reaction solvent used, but it is generally in the range of 1 to 50 percent by weight, and preferably in the range of 5 to 30 percent by weight.

The temperature for the sulfonylation depends on the types of sulfonylating agent and reaction solvent used, but it is generally between the freezing point and the boiling point of the reaction solvent. In order to complete the reaction in short time, the reaction is performed at a higher temperature; in order to suppress side reactions, the reaction is performed at a lower temperature. The temperature is generally in the range of -20 to 100°C, and more preferably in the range of -10 to 30°C.

The reaction time for the sulfonylation depends on the types of sulfonylating agent and reaction solvent used and reaction temperature, but it is generally in the range of 1 to 24 hours at a reaction temperature in the range of -10 to 30°C.

After the sulfonylation, the sulfonylating agent is quenched with water or an acid solution, such as that of hydrochloric acid or sulfuric acid. For a crystalline compound, such as a tosyl compound, however, the targeted compound can be simply obtained by filtration because the compound can be precipitated by adding water to the reaction solution. Otherwise, after the organic phase is separated, the organic phase is washed several times with water or an acid solution to remove a base, and the solvent is evaporated under reduced pressure. Thus, the optically active 7-substituted-2-sulfonyloxytetralin (3) is obtained. The optically active 7-substituted-2-sulfonyloxytetralin (3) may be purified by silica gel chromatography, recrystallization, or the like if necessary.

In the subsequent step, a nitrogen nucleophile is allowed to act on the optically active 7-substituted-2-sulfonyloxytetralin (3) to produce an optically active 2,7-substituted tetralin (4). In this instance, the configuration of the compound is reversed from an (R) form to an (S) form or from an (S) form to an (R) form. It is preferred that the (S) form of optically active 2,7-substituted tetralin (4) is produced from the (R) form of optically active 7-substituted-2-sulfonyloxytetralin (3).

X of the optically active 2,7-substituted tetralin (4) expressed by general formula (4) represents a non-substituted amino group, an alkylamino group having 1 to 10 carbon atoms, an arylamino group having 6 to 20 carbon atoms, an aralkylamino group having 7 to 20 carbon atoms, an amido group having 1 to 20 carbon atoms, an imido group having 2 to 20 carbon atoms, a sulfonylamino group having 1 to 20 carbon atoms, or an azido group. Specifically, exemplary groups include the amino group; alkylamino groups having 1 to 10 carbon atoms, such as methylamino, ethylamino, and dimethylamino; aryl amino groups having 6 to 20 carbon atoms, such as phenylamino; aralkylamino groups having 7 to 20 carbon atoms, such as benzylamino; amido groups having 1 to 20 carbon atoms, such as acetylamino, diacetylamino, and diformylamino; imido groups having 2 to 20 carbon atoms, such as phthalimido; sulfonylamino groups having 1 to 20 carbon atoms, such as benzenesulfonylamino, p-nitrobenzenesulfonylamino, o-nitrobenzenesulfonylamino, m-nitrobenzenesulfonylamino, and p-toluenesulfonylamino; and the azido group. Among these groups, amino, phthalimido, and azido groups are preferable from the viewpoint of increase in reaction yield and ease of transformation into the amino group.

Exemplary nitrogen nucleophiles used for this reaction include amines, such as ammonia, methylamine, dimethylamine, benzylamine, and aniline; amides, such as acetylamide; imides, such as diacetylimide, diformylimide, phthalimide, and metal salts of phthalimide; sulfonylamides, such as benzenesulfonylamide, p-nitrobenzenesulfonylamide, o-nitrobenzenesulfonylamide, m-nitrobenzenesulfonylamide, and p-toluenesulfonylamide; and metal azides, such as sodium azide. Among these nucleophiles, ammonia, metal azides, and alkali metal salts of phthalimide are preferable from the viewpoint of increase in reaction yield and ease of transformation into the amino group. In particular, if ammonia is used as the nitrogen nucleophile, X of the product is the amino group, and the product is identical with the optically active 7-substituted-2-aminotetralin expressed by general formula (5). Therefore, it is unnecessary to perform the step of transforming X into the amino group, as a matter of course.

In the process of the present invention, it is preferable that the nitrogen nucleophile is a metal azide and the X is an azido group, and that the 2,7-substituted tetralin expressed by general formula (4) is transformed into the optically active 7-substituted-2-aminotetralin expressed by general formula (5) or its salt by reduction. In this reduction, hydrogen is preferably used.

The amount of nitrogen nucleophile depends on the types of nitrogen nucleophile and solvent used. Normally, 1 mole equivalent or more of nitrogen nucleophile is used relative to the optically active 7-substituted-2-sulfonyloxytetralin (3). The preferred amount is generally 10.0 mole equivalents or less, more preferably 5.0 mole equivalents or less, and still more preferably 2.0 mole equivalents or less, from the viewpoint of economical efficiency. In particular, if ammonia is used as the nitrogen nucleophile, the amount of the nitrogen nucleophile is preferably 10 mole equivalents or more, and more preferably 50 mole equivalents or more, from the viewpoint of increasing the yield. In view of economical efficiency, the amount is preferably 300 mole equivalents or less, and more preferably 200 mole equivalents or less.

The reaction solvent used for the substitution is not particularly limited as long as it does not inhibit the reaction. Exemplary solvents include: hydrocarbon solvents, such as pentane, hexane, heptane, cyclohexane; and petroleum ether; esters, such as ethyl acetate, methyl acetate, propyl acetate, and methyl propionate; alcohols, such as methanol, ethanol, isopropanol, 1-butanol, and 2-butanol; aromatic hydrocarbons, such as toluene, benzene, and xylene; nitriles, such as acetonitrile and propionitrile; ethers, such as tert-butyl methyl ether, diethyl ether, diisopropyl ether, tetrahydrofuran, dioxane, and dimethoxyethane; ketones, such as acetone and ethyl methyl ketone; amides, such as N,N-dimethylformamide and N,N-dimethylacetamide; sulfoxides, such as dimethylsulfoxide; halogenated hydrocarbons, such'as methylene chloride, 1,2-dichloroethylene, chloroform, and carbon tetrachloride; amines, such as pyridine and triethylamine; and water. These solvents may be used singly or in combination. Among these solvents, preferred solvents are methanol, ethanol, isopropanol, 1-butanol, 2-butanol, toluene, acetonitrile, tetrahydrofuran, dimethoxyethane, N,N-dimethylformamide, triethylamine, and mixtures containing at least two of these solvents from the viewpoint of yield. If a mixed solvent is used, the mixing ratio is not particularly limited. In use of ammonia as the nitrogen nucleophile, the substitution can proceed even without a reaction solvent.

The concentration of the optically active 7-substituted-2-sulfonyloxytetralin (3) in the substitution reaction depends on the reaction solvent used, but it is generally in the range of 1 to 50 percent by weight, and preferably in the range of 5 to 30 percent by weight.

The temperature for the substitution reaction depends on the types of nitrogen nucleophile and reaction solvent used, but it is generally between the freezing point and the boiling point of the reaction solvent. In order to complete the reaction in short time, the reaction is performed at a higher temperature; in order to suppress side reactions, the reaction is performed at a lower temperature. The temperature is generally in the range of 20 to 200°C, and more preferably in the range of 50 to 150°C.

The reaction time for the substitution reaction depends on the types of nitrogen nucleophile and reaction solvent used and reaction temperature, but it is generally in the range of 1 to 24 hours at a reaction temperature in the range of 50 to 150°C.

If a nonaqueous solvent such as toluene or 1-butanol is used, after the substitution reaction, the organic phase is washed several times with a neutral or alkaline aqueous solution, and the solvent is evaporated under reduced pressure to obtain the optically active 2,7-substituted tetralin (4). If a water-soluble solvent is used, such as methanol, dimethylformamide, tetrahydrofuran, or acetonitrile, the solvent is evaporated under reduced pressure, and the product is then dissolved in an organic solvent, such as ethyl acetate or toluene. After washing the solution in the same manner, the solvent is evaporated under reduced pressure to obtain the optically active 2,7-substituted tetralin (4).

The substitution reaction often produces a β-eliminated 3,4-dihydro-7-substituted naphthalene as a byproduct. However, this compound is oily in many cases. Accordingly, if the optically active 2,7-substituted tetralin (4) is solid, the byproduct can be easily removed by crystallization. The optically active 2,7-substituted tetralin (4) may be purified by silica gel chromatography or the like.

If the nitrogen nucleophile is ammonia, the targeted compound, optically active 7-substituted-2-aminotetralin (5) is produced, as described above. The resulting compound (5) can be generally extracted into an organic phase in the presence of an organic solvent and water at a pH from neutral to basic (pH 7 or more). The organic solvent is, for example, toluene or ethyl acetate.

In order to remove impurities, the solution after the reaction or the above-described extract may be neutralized with an acid to extract a salt of the optically active 7-substituted-2-aminotetralin (5) with the acid into a water phase. The water phase is washed with an organic solvent, such as toluene or ethyl acetate. The resulting salt of the optically active 7-substituted-2-aminotetralin (5) with the acid may be obtained in a solution or in a crystalline form. The salt can be converted into a free amine and extracted with the above-described solvent to obtain an optically active 7-substituted-2-aminotetralin (5) as a free amine.

The resulting extract is, for example, heated under reduced pressure to evaporate the reaction solvent and the extractant, and thus the optically active 7-substituted-2-aminotetralin (5) is obtained. The optically active 7-substituted-2-aminotetralin (5) thus produced is substantially pure, but it may be purified to further increase the purity by a conventional process, such as crystallization, distillation, or column chromatography.

For crystallizing the optically active 7-substituted-2-aminotetralin (5), normally, a salt of the compound with an acid is crystallized and isolated in a crystalline form. Preferably, the acid is, but not limited to, a mineral acid, such as sulfuric acid, hydrogen chloride, or perchloric acid; or an organic acid, such as methanesulfonic acid or p-toluenesulfonic acid, and particularly preferred acid is hydrogen chloride.

The crystallization of the salt of the compound (5) with the acid is generally performed in the presence of a solvent. Exemplary crystallization solvents include alcohols, such as methanol, ethanol, and propanol; and water. Using these solvents helps efficiently remove impurities, such as optical isomers, the starting materials of the above-described reactions, and compounds having a similar structure and/or a coloring substance. These solvents may be used singly or in combination. In order to increase the yield in the crystallization, an auxiliary solvent may be used in combination. Exemplary auxiliary solvents include aromatic hydrocarbons, such as benzene, toluene, xylene, ethylbenzene, and chlorobenzene; esters, such as ethyl acetate, propyl acetate, and butyl acetate; ethers, such as diethyl ether and t-butyl methyl ether; nitriles, such as acetonitrile; aliphatic hydrocarbons, such as hexane, pentane, heptane, cyclohexane, and methylcyclohexane.

For crystallizing the salt of the optically active 7-substituted-2-aminotetralin (5) with an acid, the acid is added to the extract of the compound (5) or a mixture of the extract and the above-described solvent to form the salt of the compound (5) with the acid, and the salt is crystallized. Alternatively, the salt of the compound (5) with the acid is recrystallized in the presence of the solvent.

As described above, the use of ammonia as the nitrogen nucleophile directly produces the optically active 7-substituted-2-aminotetralin (5). However, use of other nitrogen nucleophiles requires the step of transforming the substituent X of the optically active 2,7-substituted tetralin (4) into the amino group. The reaction process in this step depends on the type of nitrogen nucleophile used, as a matter of course.

In use of a metal salt of phthalimide as the nitrogen nucleophile, the substituent X of the optically active 7,2-substituted tetralin (4) is the phthalimide group. The phthalimide group is transformed into the amino group by deprotection to obtain the optically active 7-substituted-2-aminotetralin (5). The deprotection may be performed by hydrolysis using hydrochloric acid, sulfuric acid, or the like, or by adding hydrazine.

In use of a metal azide as the nitrogen nucleophile, the substituent X of the optically active 7,2-substituted tetralin (4) is the azido group. The azido group is transformed into the amino group by reduction to obtain the optically active 7-substituted-2-aminotetralin (5). This reduction reaction will now be described below, but it is not limited to this.

Exemplary reducing agents used in this reduction include hydrogen-type agents, such as hydrogen and ammonium formate; phosphorus-type agents, such as triphenylphosphine and trimethyl phosphite; hydride agents, such as sodium borohydride, borane, and lithium aluminiumhydride.

If hydrogen is used as the reducing agent, a transition metal catalyst is necessary. Such transition metal catalysts include palladium/carbon, palladium black, palladium oxide, palladium/calcium carbonate, platinum, and platinum oxide.

The amount of the transition metal catalyst depends on the type of transition metal used. Normally, 1 percent by weight or more of the catalyst is used relative to the optically active 2,7-substituted tetralin (4). As the amount is increased, the reaction rate increases. However, the preferred amount is generally 100 percent by weight or less, more preferably 30 percent by weight or less, and still more preferably 10 percent by weight or less, from the viewpoint of economical efficiency.

The hydrogen pressure is set between, for example, normal pressure and 10 kg/cm². Although the pressure may be set high to increase the reaction rate if necessary, the reaction often proceeds rapidly at normal pressure.

The reaction solvent used for the reduction reaction is not particularly limited as long as it does not inhibit the reaction. Exemplary solvents include: hydrocarbons, such as pentane, hexane, heptane, cyclohexane, and petroleum ether; esters, such as ethyl acetate, methyl acetate, propyl acetate, and methyl propionate; alcohols, such as methanol, ethanol, isopropanol, and 1-butanol; aromatic hydrocarbons, such as toluene, benzene, and xylene; nitriles, such as acetonitrile and propionitrile; ethers, such as tert-butyl methyl ether, diethyl ether, diisopropyl ether, tetrahydrofuran, dioxane, and dimethoxyethane; ketones, such as acetone and ethyl methyl ketone; amides, such as N,N-dimethylformamide and N,N-dimethylacetamide; sulfoxides, such as dimethylsulfoxide; halogenated hydrocarbons, such as methylene chloride, 1,2-dichloroethylene, chloroform, and carbon tetrachloride; and water. These solvents may be used singly or in combination. Among these solvent, preferred solvents are water, methanol, ethanol, isopropanol, 1-butanol, toluene, acetonitrile, tetrahydrofuran, ethyl acetate, and mixtures containing at least two of these solvents from the viewpoint of yield. If a mixed solvent is used, the mixing ratio is not particularly limited.

The concentration of the optically active 2,7-substituted tetralin (4) in the reduction reaction depends on the reaction solvent used, but it is generally in the range of 1 to 50 percent by weight, and preferably in the range of 5 to 30 percent by weight.

The temperature for the reduction reaction depends on the types of reducing agent and reaction solvent used, but it is generally between the freezing point and the boiling point of the reaction solvent. In order to complete the reaction in short time, the reaction is performed at a higher temperature; in order to suppress side reactions, the reaction is performed at a lower temperature. The temperature is generally in the range of -20 to 150°C, and more preferably in the range of -10 to 100°C.

The reaction time for the reduction reaction depends on the types of reducing agent and reaction solvent used and reaction temperature, but it is generally in the range of 1 to 24 hours at a reaction temperature in the range of 20 to 120°C.

After the reduction, the resulting optically active 7-substituted-2-aminotetralin (5) can be obtained in the same manner as in the substitution reaction from the compound (3) to the compound (4). In addition, the compound (5) may be crystallized by forming a salt with an acid.

### Best Mode for Carrying Out the Invention

The present invention will now be further described in detail with reference to Examples, but the invention is not limited to the examples.

### (EXAMPLE 1) Preparation of (R)-7-methoxy-2-tetralol

In a 500 mL Sakaguchi flasks are placed 50 mL aliquots of 500 mL of a liquid culture medium (pH 7.0) containing 40 g of glucose, 3 g of an yeast extract, 6.5 g of diammonium hydrogen phosphate, 1 g of dipotassium hydrogen phosphate, 0.8 g of magnesium sulfate heptahydrate, 60 mg of zinc sulfate heptahydrate, 90 mg of iron sulfate heptahydrate, 5 mg of copper sulfate pentahydrate, 10 mg of manganese sulfate tetrahydrate, and 100 mg of sodium chloride (per liter each). Each aliquot was steamsterilized at 120°C for 20 minutes. A loopful of *Candida magnoliae* IF0705 was aseptically inoculated into the aliquot, followed by shaking to cultivate the microorganism at 30°C for 24 hours. After the cultivation, the culture broth was centrifuged to collect cells, and the cells were suspended in 50 mL of a 100 mM phosphate buffer solution (pH 7.0). To the suspension were added 1 g of 7-methoxy-2-tetralone and 5 g of glucose. While being stirred at 30°C for 24 hours, the reaction mixture is allowed to react with the pH maintained at 6.5 with an aqueous solution of 5 M sodium hydroxide. After the reaction had been completed, the reaction mixture was extracted two times with 500 mL of ethyl acetate. The organic phase was dried over anhydrous sodium sulfate. The anhydrous sodium sulfate was removed by filtration, and the solvent was evaporated under reduced pressure. The residue was purified by silica gel column chromatography to yield 900 mg of (R)-7-methoxy-2-tetralol (yield: 89%). The optical purity measured under the following conditions was 81% ee.

Column: Daicel Chiralcel OJ (registered trademark) (4.6 mm x 250 mm); eluant: n-hexane/isopropanol = 9/1; flow rate: 1 mL/min, detection: 210 nm; column temperature: 30°C; elution time: 11 minutes for (R)-7-methoxy-2-tetralone, 14 minutes for (S)-7-methoxy-2-tetralone.

### (EXAMPLE 2) Preparation of (R)-7-methoxy-2-tetralol

Using a culture broth of *Candida maris* IFO10003 prepared in the culture of Example 1 in precisely the same manner as in Example 1, cell reaction and extraction and purification of the product were performed in the same manner to yield 900 mg of (R)-7-methoxy-2-tetralol. The optical purity was 76% ee.

### (EXAMPLE 3) Preparation of (R)-7-methoxy-2-methanesulfonyloxytetralin

(R)-7-methoxy-2-tetralol (87.8% ee) was separately synthesized according to Example 1, and 1.00 g of this compound was dissolved in 6 mL of methylene chloride. While this solution is cooled with ice, 1.35 g of methanesulfonyl chloride and 1.42 g of triethylamine were added to the solution, and the mixture was stirred at the same temperature for 2 hours. After being washed twice with 1 M hydrochloric acid, the reaction mixture was washed with a saturated aqueous solution of sodium hydrogen carbonate and dried over anhydrous sodium sulfate. Then, the solvent was evaporated under reduced pressure to yield 1.31 g of oil. The oil was purified with a silica gel column (hexane/ethyl acetate = 1/1) to yield 1.06 g of the compound of the heading (yield: 74%).
¹H-NMR (CDCl₃) δ ppm: δ 2.10-2.20 (q, 2H), 2.76-3.02 (m, 2H), 3.02 (s, 3H), 3.00-3.23 (m, 2H), 3.78 (S, 3H), 5.18 (m, 1H), 6.60 (s, 1H), 6.73 (d, 1H), 7.02 (d, 1H).

### (EXAMPLE 4) Preparation of (R)-7-methoxy-2-p-toluenesulfonyloxytetralin

In 19 mL of pyridine was dissolved 1.01 g of (R)-7-methoxy-2-tetralol (87.8% ee). While the solution is cooled with ice, 2.42 g of p-toluenesulfonyl chloride was added to the solution. The reaction mixture was allowed to stand at -20°C for 16 hours and at 0° C for 20 hours. Ice-cold water was added to the reaction mixture, followed by stirring for 30 minutes. The product was extracted three times with ethyl acetate. After being washed five times with 1 M hydrochloric acid, the organic phase was washed with a saturated aqueous solution of sodium chloride and dried over anhydrous sodium sulfate. Then, the solvent was evaporated under reduced pressure to yield 1.84 g of oil. The oil was purified with a silica gel column (hexane/ethyl acetate = 6/4) to yield 1.34 g of the compound of the heading (yield: 72%).
¹H-NMR (CDCl₃) δ ppm: δ 2.00 (q, 2H), 2.46 (s, 3H), 2.65-3.02 (m, 4H), 3.78 (S, 3H), 4.93 (m, 1H), 6.50 (s, 1H), 6.70 (d, 1H), 6.96 (d, 1H), 7.35 (d, 2H), 7.80 (d, 2H).

### (EXAMPLE 5) Preparation of (R)-7-methoxy-2-m-nitrobenzenesulfonyloxytetralin

In 19 mL of pyridine was dissolved 1.01 g of (R)-7-methoxy-2-tetralol (87.8% ee). While the solution was cooled with ice, 2.53 g of m-nitrobenzenesulfonyl chloride was added to the solution. The reaction mixture was allowed to stand at -20°C for 16 hours and at 0° C for 20 hours. Ice-cold water was added to the reaction mixture, followed by stirring for 30 minutes. The product was extracted three times with ethyl acetate. After being washed five times with 1 M hydrochloric acid, the organic phase was washed with a saturated aqueous solution of sodium chloride and dried over anhydrous sodium sulfate. Then, the solvent was evaporated under reduced pressure to yield 1.25 g of yellow solid. The solid was purified with a silica gel column (hexane/ethyl acetate = 7/3) to yield 0.46 g of the compound of the heading (yield: 22%).
¹H-NMR (CDCl₃) δ ppm: δ 2.00-2.15 (m, 2H), 2.70-3.10 (m, 4H), 3.75 (S, 3H), 5.12 (m, 1H), 6.46 (s, 1H), 6.70 (d, 1H), 6.98 (d, 1H), 7.80 (t, 1H), 8.22 (d, 1H), 8.50 (d, 1H), 8.72 (s, 1H).

### (EXAMPLE 6) Preparation of (S)-7-methoxy-2-aminotetralin

In a 10 mL autoclave placed were 253.1 mg of (R)-7-methoxy-2-methanesulfonyloxytetralin (87.8% ee) separately synthesized according to Example 3 and 1.3 mL of methanol. After cooling the mixture to -78°C, 2.37 g (139 equivalents) of liquid ammonia was added and the autoclave was sealed. The reaction mixture was stirred at 95°C (external temperature) for 6 hours, and then the autoclave was opened at -78°C. After evaporating ammonia under reduced pressure at room temperature, the pH of the reaction mixture was adjusted to 3 with 3M hydrochloric acid, and then methanol was evaporated under reduced pressure. After adding ethyl acetate to the residue, the product was extracted two times with 1 M hydrochloric acid. The extract was adjusted to pH 11 with a 30% sodium hydroxide solution and further subjected to extraction with ethyl acetate. The organic phase was washed with a saturated aqueous solution of sodium chloride and dried over anhydrous sodium sulfate. Then, the solvent was evaporated under reduced pressure to yield 74 mg of the compound of the heading (yield: 42%; optical purity: 87.0% ee). The optical purity was determined by high performance liquid chromatography (HPLC) after deriving methylcarbamate.
HPLC conditions:
Column used, Chiralcel OD
Mobile phase, hexane/isopropanol = 9/1
Temperature, 30°C; measuring wavelength, 254 nm; flow rate, 1.0 mL/min
¹H-NMR (CDCl₃) δ ppm: δ 1.50-1.65 (m, 1H), 1.95-2.05 (m, 1H), 2.50-2.60 (m, 1H), 2.72-2.90 (m, 2H), 2.93-3.05 (m, 1H), 3.15-3.25 (m, 1H), 3.78 (S, 3H), 6.60 (s, 1H), 6.70 (d, 1H), 7.00 (d, 1H).

### (EXAMPLES 7 to 17) Preparation of (S)-7-methoxy-2-aminotetralin

In a 10 mL autoclave placed were 128 mg of (R)-7-methoxy-2-methanesulfonyloxytetralin (87.8% ee) and 0.7 mL of a solvent listed in Table 1. After cooling the mixture to -78°C, liquid ammonia was added in an amount shown in Table 1 and the autoclave was sealed. The reaction mixture was stirred at a temperature shown in Table 1 for 6 hours, and then the autoclave was opened at -78°C. After evaporating ammonia under reduced pressure at room temperature, the pH of the reaction mixture was adjusted to 3 with 3 M hydrochloric acid, and then methanol was evaporated under reduced pressure. After adding ethyl acetate to the residue, the product was extracted two times with 1 M hydrochloric acid. The extract was adjusted to pH 11 with a 30% sodium hydroxide solution and further subjected to extraction with ethyl acetate. The organic phase was washed with a saturated aqueous solution of sodium chloride and dried over anhydrous sodium sulfate. Then, the solvent was evaporated under reduced pressure to yield the compound of the heading in an amount shown in Table 1. The yield is shown in Table 1.

**Table 1**

| Example | Solvent | NH₃ (equivalent) | Temperature (°C) | Yield |
|---|---|---|---|---|
| **7** | **MeOH** | **166** | **150** | **46%** |
| **8** | **MeOH** | **263** | **95** | **41%** |
| **9** | NaOH/toluene = 1/1 | **164** | **95** | **53%** |
| **10** | NaOH/water = 1/1 | **164** | **95** | **37%** |
| **11** | **nBuOH** | **177** | **95** | **53%** |
| **12** | Toluene | **156** | **100** | **47%** |
| **13** | **DMF** | **168** | **75** | **50%** |
| **14** | **THF** | **210** | **100** | **51%** |
| **15** | **DME** | **200** | **100** | **51%** |
| **16** | Acetonitrile | **164** | **100** | **50%** |
| **17** | Triethylamine | **162** | **100** | **51%** |

### (EXAMPLE 18) Preparation of (S)-7-methoxy-2-aminotetralin

In a 10 mL autoclave placed were 165.3 mg of (R)-7-methoxy-2-p-toluenesulfonyloxytetralin (87.8% ee) and 0.7 mL of methanol. After cooling the mixture to -78°C, 1.49 g (176 equivalents) of liquid ammonia was added and the autoclave was sealed. The reaction mixture was stirred at 95°C (external temperature) for 6 hours, and then the autoclave was opened at -78°C. After evaporating ammonia under reduced pressure at room temperature, the pH of the reaction mixture was adjusted to 3 with 3 M hydrochloric acid, and then methanol was evaporated under reduced pressure. After adding ethyl acetate to the residue, the product was extracted two times with 1 M hydrochloric acid. The extract was adjusted to pH 11 with a 30% sodium hydroxide solution and further subjected to extraction with ethyl acetate. The organic phase was washed with a saturated aqueous solution of sodium chloride and dried over anhydrous sodium sulfate. Then, the solvent was evaporated under reduced pressure to yield 42 mg of the compound of the heading (yield: 48%).

### (EXAMPLE 19) Preparation of (S)-7-methoxy-2-aminotetralin

In a 10 mL autoclave placed were 181.0 mg of (R)-7-methoxy-2-(3-nitrobenzenesulfonyloxy)tetralin (87.8% ee) and 0.7 mL of methanol. After cooling the mixture to -78°C, 1.49 g (176 equivalents) of liquid ammonia was added and the autoclave was sealed. The reaction mixture was stirred at 95°C (external temperature) for 6 hours, and then the autoclave was opened at -78°C. After evaporating ammonia under reduced pressure at room temperature, the pH of the reaction mixture was adjusted to 3 with 3 M hydrochloric acid, and then methanol was evaporated under reduced pressure. After adding ethyl acetate to the residue, the product was extracted two times with 1 M hydrochloric acid. The extract was adjusted to pH 11 with a 30% sodium hydroxide solution and further subjected to extraction with ethyl acetate. The organic phase was washed with a saturated aqueous solution of sodium chloride and dried over anhydrous sodium sulfate. Then, the solvent was evaporated under reduced pressure to yield 28 mg of the compound of the heading (yield: 32%).

### (EXAMPLE 20) Preparation of (S)-7-methoxy-2-aminotetralin hydrochloride

Ethanol in an amount of 18.4 mL was added to 184 g of a mixed solution of (S)-7-methoxy-2-aminotetralin (18.4 g, optical purity: 99.8% ee) separately synthesized according to Example 6 and toluene. While the mixture was being stirred, 11.9 g of concentrated hydrochloric acid was added at an internal temperature of 25°C over a period of 1.5 hours. The mixture was stirred at 25°C for another one hour, and then precipitated crystals were filtrated. The collected wet crystals were washed twice with 37 mL of toluene and dried to yield the crystals of (S)-7-methoxy-2-aminotetralin hydrochloride (17.6 g; yield: 83%; optical purity: 99.9% ee).

### (EXAMPLE 21) Preparation of (S)-7-methoxy-2-aminotetralin hydrochloride

To (S)-7-methoxy-2-aminotetralin hydrochloride (20.6 g, optical purity: 99.9% ee) were added 20 mL of ethanol, 180 mL of toluene, and 7 mL of water, and the mixture was heated to dissolve the crystals. After stirring for 15 minutes, the solution was cooled to room temperature, and further cooled with ice. After stirring for 30 minutes, the crystals were filtrated. The collected wet crystals were washed twice with 40 mL of toluene, and dried to yield the crystals of (s)-7-methoxy-2-aminotetralin hydrochloride (19.0 g; yield: 92%; optical purity: 99.9% ee).

### (EXAMPLE 22) Preparation of (S)-7-methoxy-2-aminotetralin hydrochloride

Concentrated hydrochloric acid in an amount of 63 mg was added to a mixed solution of separately synthesized (S)-7-methoxy-2-aminotetralin (99.2 mg; optical purity: 91.2% ee) and 1 mL of ethanol at an internal temperature of 25°C while the mixture was being stirred. The mixture was stirred at 25°C for another one hour, and then precipitated crystals were filtrated. The collected wet crystals were washed with toluene and dried to yield the crystals of (S)-7-methoxy-2-aminotetralin hydrochloride (76.2 mg; yield: 64%; optical purity: 95.5% ee).

### (EXAMPLE 23) Preparation of (S)-7-methoxy-2-aminotetralin hydrochloride

An isopropanol solution of hydrogen chloride (hydrogen chloride concentration: 34%) in an amount of 65 mg was added to a mixed solution of (S)-7-methoxy-2-aminotetralin (102.1 mg; optical purity: 91.2% ee) and 1 mL of isopropanol at an internal temperature of 25°C while the mixture was being stirred. The mixture was stirred at 25°C for another one hour, and then precipitated crystals were filtrated. The collected wet crystals were washed with isopropanol and dried to yield the crystals of (S)-7-methoxy-2-aminotetralin hydrochloride (76.2 mg; yield: 63%; optical purity: 98.7% ee).

### (EXAMPLE 24) Preparation of (S)-7-methoxy-2-aminotetralin hydrochloride

The mixture of separately synthesized (S)-7-methoxy-2-aminotetralin hydrochloride (100.7 mg; optical purity: 93.0% ee) and 1 mL of ethanol was heated to dissolve. The solution was cooled to room temperature while being stirred. After being stirred for 1 hour, the solution was stirred in an ice bath for another one hour. The precipitated crystals were filtrated. The collected wet crystals were washed with toluene and dried to yield the crystals of (s)-7-methoxy-2-aminotetralin hydrochloride (69.2 mg; yield: 69%; optical purity: 99.3% ee).

### (EXAMPLE 25) Preparation of (S)-7-mgthoxy-2-aminotetralin hydrochloride

A mixture of (S)-7-methoxy-2-aminotetralin hydrochloride (100.4 mg; optical purity: 93.0% ee) and 1 mL of hydrated ethanol (ethanol:water = 95:5 (on a volume basis)) was heated to dissolve. The solution was cooled to room temperature while being stirred, and further stirred for 0.5 hour. The precipitated crystals were filtrated. The collected wet crystals were washed with toluene and dried to yield the crystals of (s)-7-methoxy-2-aminotetralin hydrochloride (50.6 mg; yield: 50.4%; optical purity: 98.5% ee).

### (EXAMPLE 26) Preparation of (S)-7-methoxy-2-aminotetralin hydrochloride

The mixture of (S)-7-methoxy-2-aminotetralin hydrochloride (100.6 mg; optical purity: 93.0% ee) and 5 mL of isopropanol was heated to dissolve. The solution was cooled to room temperature while being stirred. After being stirred for 0.5 hour, the solution was stirred in an ice bath for another one hour. The precipitated crystals were filtrated. The collected wet crystals were washed with isopropanol and dried to yield the crystals of (s)-7-methoxy-2-aminotetralin hydrochloride (51.0 mg; yield: 51%; optical purity: 99.3% ee).

### (EXAMPLE 27) Preparation of (S)-7-methoxy-2-aminotetralin hydrochloride

The mixture of (S)-7-methoxy-2-aminotetralin hydrochloride (100.6 mg; optical purity: 93.0% ee) and 1 mL of ethanol was heated to dissolve. To the solution was added 1 mL of diethyl ether while being stirred. The solution was cooled to room temperature and stirred for 0.5 hour. The precipitated crystals were filtrated. The collected wet crystals were washed with diethyl ether and dried to yield the crystals of (s)-7-methoxy-2-aminotetralin hydrochloride (85.4 mg; yield: 85%; optical purity: 98.4% ee).

### (EXAMPLE 28) Preparation of (S)-7-methoxy-2-azidotetralin

DMSO in an amount of 15.0 mL was added to (R)-7-methoxy-2-methanesulfonyloxytetralin (261 mg, 1.018 mmol; optical purity: 87.8% ee) separately synthesized according to Example 3, and the reactor was purged with nitrogen. NaN₃ (purity: 90%; 740.3 mg, 10.20 mmol, 10 equivalents) was added at room temperature, followed by stirring at 50°C for 4 hours. After cooling, 15 mL of water was added, and the product was extracted two times with 20 mL of toluene. The organic phase was washed with 20 mL of water and 20 mL of brine and concentrated to yield 195 mg of crude product. According to ¹H-NMR, the crude product contained 92% of (S)-7-methoxy-2-azidotetralin and 8% of 7-methoxy-3,4-dihydronaphthalene (yield of 7-methoxy-2-azidotetralin: 88%).
¹H-NMR (CDCl₃) δ ppm: 1.83-1.91 (m, 1H), 2.08-2.11 (m, 1H), 2.73-2.92 (m, 3H), 3.05 (dd, J = 5.0, 16.4 Hz, 1H), 3.77 (s, 3H), 3.78-3.88 (m, 1H), 6.61 (d, J = 2.4 Hz, 1H), 6.72 (dd, J = 2.4, 8.3 Hz, 1H), 7.01 (d, J = 8.3 Hz, 1H), 7.25 (s, 1H).

### (EXAMPLE 29) Preparation of (S)-7-methoxy-2-aminotetralin

A toluene solution containing 2.66 g of (S)-7-methoxy-2-azidotetralin prepared in Example 28 was cooled to 5°C, and then 0.27 g (10 percent by weight relative to the substrate) of hydrous Pd/C (water content: 50 percent by weight) was added in an atmosphere of nitrogen. Then, the atmosphere in the vessel was replaced with a hydrogen atmosphere and the reaction mixture was stirred for 8 hours without changing temperature. After the reaction had been completed, the temperature is increased to room temperature, and the Pd/C was removed by filtration under reduced pressure. The removed Pd/C was washed with 13 g of toluene. Thus, a toluene solution containing 2.17 g of (S)-7-methoxy-2-aminotetralin was obtained (yield: 93%).

### (EXAMPLE 30) Preparation of (S)-7-methoxy-2-phthalimidotetralin

Phthalimide potassium salt (281 mg, 1.5 equivalents) was added to (R)-7-methoxy-2-methanesulfonyloxytetralin (265 mg, 1.03 mmol; optical purity: 87.8% ee) and 5 mL of DMF, and the reactor was purged with nitrogen. The reaction mixture was heated to 60°C and allowed to react for 24 hours. After cooling, 15 mL of water was added, and the product was extracted two times with 20 mL of toluene. The organic phase was washed with 20 mL of water and 20 mL of brine and concentrated to yield 410 mg of crude product. The crude product was purified by silica gel column chromatography (eluent: hexane/ethyl acetate = 2:1) to yield 85.4 mg of (S)-7-methoxy-2-phthalimidotetralin (yield: 27%).
¹H-NMR (CDCl₃) δ ppm: 1.95-2.03 (m, 1H), 2.63-2.94 (m, 3H), 3.62 (dd, J = 12.0, 16.5 Hz, 1H), 3.79 (s, 3H), 4.58-4.63 (m, 1H), 6.63 (d, J = 2.4 Hz, 1H), 6.75 (dd, J = 2.4, 8.3 Hz, 1H), 7.05 (d, J = 8.3 Hz, 1H), 7.25 (s, 1H), 7.65-7.78 (m, 2H), 7.82-7.89 (m, 2H).

### (EXAMPLE 31) Preparation of (R)-7-methoxy-2-tetralol

In a test tube was placed 5 mL of liquid culture medium (pH 7.0) having the composition described in Example 1, and was steamsterilized at 120°C for 20 minutes. A loopful of one of the microorganism listed in Table 2 was aseptically inoculated into the culture, followed by shaking to cultivate the microorganism at 30°C for 24 to 72 hours. After the cultivation, the culture broth was centrifuged to collect cells, and the cells were suspended in 1 mL of a 100 mM phosphate buffer solution (pH 7.0). To this suspension were added 5 mg of 7-methoxy-2-tetralone and 5 g of glucose, followed by stirring at 30°C for 24 hours. After reaction, 5 mL of ethyl acetate was added to extract the product. The organic phase was analyzed by high performance liquid chromatography and measured the yield and the optical purity of the product (R)-7-methoxy-2-tetralol. The results are shown in Table 2. The chromatography for calculating the yield was performed under the following conditions, and the optical purity was measured in the same manner as in Example 1.
Column: Nomura Chemical, Develosil ODS-HG3 (4.6 mm x 150 mm); eluent: water/acetonitrile = 2/1; flow rate: 0.7 mL/min; detection: 254 nm; column temperature: room temperature.

**Table 2**

| Microorganism | | Yield (%) | Optical purity (% ee) |
|---|---|---|---|
| *Candida catenulata* | IFO 0745 | 14.9 | 33.1 |
| *Candida glabrata* | IFO 0005 | 28.0 | 70.8 |
| *Candida maltosa* | IFO 1976 | 29.7 | 70.3 |
| *Candida maris* | IFO 10003 | 41.1 | 34.2 |
| *Candida albicans* | IFO 1594 | 68.3 | 61.7 |
| *Candida fennica* | CBS 6087 | 58.4 | 73.1 |
| *Debaryomyces hansenii var. hansenii* | IFO 0019 | 10.6 | 53.1 |
| *Pichia anomala* | IFO 0118 | 44.6 | 52.5 |
| *Kluyveromyces polysporus* | IFO 0996 | 90.9 | 35.7 |
| *Metschnikowia bicuspidata var, bicuspidata* | IFO 1408 | 76.1 | 49.9 |
| *Ogataea minuta var. nonfermentans* | IFO 1473 | 30.6 | 52.5 |
| *Sporidiobolus johnsonii* | IFO 6903 | 31.8 | 88.1 |
| *Torulaspora delbrueckii* | IFO 0381 | 19.5 | 47.7 |
| *Geotrichum fermentans* | IFO 1199 | 31.2 | 36.1 |
| *Yamadazyma farinosa* | IFO 0534 | 49.0 | 79.7 |

### (EXAMPLE 32) Preparation of (S)-7-methoxy-2-tetralol

Using the microorganisms listed in Table 3, the same operation as Example 31 was performed to prepare (S)-7-methoxy-2-tetralol. The results are shown in Table 3.

**Table 3**

| Microorganism | | Yield (%) | Optical purity (% ee) |
|---|---|---|---|
| *Candida glaebosa* | IFO 1353 | 79.7 | 61.7 |
| *Candida haemulonii* | IFO 10001 | 25.8 | 88.5 |
| *Candida holmii* | IFO 0660 | 34.7 | 37.3 |
| *Candida intermedia* | IFO 0761 | 23.4 | 79.5 |
| *Candida boidinii* | IFO10240 | 14.5 | 39.1 |
| *Candida pintolopesii* | IFO 0729 | 31.4 | 36.6 |
| *Candida oleophila* | IFO 1021 | 67.3 | 71.6 |
| *Candida sonorensis* | IFO 10027 | 16.5 | 32.3 |
| *Candida tropicalis* | IFO 0618 | 65.2 | 52.9 |
| *Debaryomyces carsonii* | IFO 0946 | 46.1 | 83.1 |
| *Endomyces decipiens* | IFO 0102 | 16.8 | 79.3 |
| *Dipodascus ovetensis* | IFO 1201 | 55.6 | 93.9 |
| *Saccharomycopsis selenospora* | IFO 1850 | 56.1 | 44.7 |
| *Issatchenkia terricola* | IFO 0933 | 16.0 | 76.9 |
| *Kitraishia capsulata* | IFO 0721 | 82.5 | 71.3 |
| *Lipomyces starkeyi* | IFO 0678 | 12.4 | 86.6 |
| *Lodderomyces elongisporus* | IFO 1676 | 38.8 | 61.5 |
| *Metschnikowia gruessii* | IFO 0749 | 72.4 | 36.5 |
| *Pichia wickerhamii* | IFO 1278 | 36.9 | 69.1 |
| *Rhodosporidium toruloides* | IFO 0559 | 23.9 | 30.9 |
| *Rhodotorula araucariae* | IFO 10053 | 16.3 | 32.5 |
| *Sporobolomyces salmonicolor* | IFO 1038 | 42.5 | 35.5 |
| *Sporidiobolus holsaticus* | IFO 1032 | 25.2 | 54.9 |
| *Debaryomyces occidentalis var.occidentalis* | IFO 0371 | 20.5 | 58.2 |
| *Saturnispora dispora* | IFO 0035 | 21.2 | 70.9 |
| *Candida stellata* | IFO 0703 | 21.6 | 54.7 |
| *Zygosaccharomyces bailii* | IFO 0519 | 27.8 | 51.4 |
| *Zygosaccharomyces bailii* | IFO 0488 | 34.5 | 44.5 |
| *Zygosaccharomyces bailii* | IFO 0493 | 34.2 | 49.3 |

### (EXAMPLE 33) Preparation of (R)-7-methoxy-2-tetralol

In a test tube was placed 5 mL of liquid culture medium (pH 7.0) containing 10 g of polypeptone, 10 g of meat extract, and 5 g of yeast extract, and was steamsterilized at 120°C for 20 minutes. A loopful of one of the microorganism listed in Table 4 was aseptically inoculated into the culture medium, followed by shaking to cultivate the microorganism at 30°C for 24 hours. After cultivation, the culture broth was centrifuged to collect cells and the cells were suspended in 1 mL of a 100 mM phosphate buffer solution (pH 7.0). To this suspension were added 5 mg of 7-methoxy-2-tetralone and 5 g of glucose, followed by stirring at 30°C for 24 hours. After reaction, 5 mL of ethyl acetate was added to extract the product. The yield and the optical purity of the product (R)-7-methoxy-2-tetralol were measured in the same manner as in Example 31. The results are shown in Table 4.

**Table 4**

| Microorganism | | Yield (%) | Optical purity (% ee) |
|---|---|---|---|
| *Arthrobacter protophormiae* | IFO 12128 | 32.4 | 57.2 |
| *Acidiphilium cryptum* | IFO 14242 | 45.3 | 89.0 |
| *Pseudomonas putida* | IFO 14164 | 26.8 | 92.1 |
| *Rhodococcus erythropolis* | IFO 12320 | 24.6 | 65.0 |
| *Devosia riboflavina* | IFO 13584 | 30.0 | 95.0 |

### (EXAMPLE 34) Preparation of (S)-7-methoxy-2-tetralol

Using the microorganisms listed in Table 5, the same operation as Example 33 was performed to prepare (S)-7-methoxy-2-tetralol. The results are shown in Table 5.

**Table 5**

| Microorganism | | Yield (%) | Optical purity (% ee) |
|---|---|---|---|
| *Cellulomonas fimi* | IFO15513 | 68.6 | 66.1 |
| *Jensenia canicruria* | IFO 13914 | 17.2 | 64.9 |
| *Micrococcus luteus* | IFO 13867 | 12.8 | 34.3 |
| *Rhodococcus erythropolis* | IAM 1474 | 66.2 | 76.4 |

### (EXAMPLE 35) Preparation of (R)-7-methoxy-2-tetralol

In a test tube was placed 5 mL of liquid culture medium (pH 7.0) having the composition described in Example 1, and was steamsterilized at 120°C for 20 minutes. A loopful of *Candida magnoliae* IFO705 was aseptically inoculated into the culture medium, followed by shaking to cultivate the microorganism at 30°C for 24 to 72 hours. After cultivation, the culture broth was centrifuged to collect cells, and the cells were suspended in 50 mL of a 100 mM phosphate buffer solution (pH 7.0). To the suspension were added 1 g of 7-methoxy-2-tetralone bisulfite adduct and 5 g of glucose. Then, the pH of the reaction mixture was maintained at 6.5 with an aqueous solution of 5 M sodium hydroxide while the solution was stirred at 30°C for 24 hours. After the reaction had been completed, the reaction mixture was extracted two times with 500 mL of ethyl acetate. The organic phase was dried over anhydrous sodium sulfate. The anhydrous sodium sulfate was removed by filtration, and the solvent was evaporated under reduced pressure. The residue was purified by silica gel column chromatography to yield 450 mg of (R)-7-methoxy-2-tetralol (yield: 45%). The optical purity measured in the same manner as in Example 1 was 81% ee.

### (REFERENCE EXAMPLE) Preparation of 7-methoxy-2-tetralone bisulfite adduct

In 200 mL of methanol was dissolved 17.6 g of 7-methoxy-2-tetralone. While the solution was cooled with ice, 100 mL of 20% sodium bisulfite solution was added to the solution, followed by stirring for 30 minutes. Precipitated white crystals were filtrated, and the product was dried under reduced pressure to yield 19 g of the compound of the heading.

### Industrial Applicability

Since the present invention includes the above-described characteristic features, an optically active 7-substituted-2-aminotetralin can be efficiently, easily, and industrially advantageously prepared from a 7-substituted-2-tetralone.

## Claims

1. A process for preparing an optically active 7-substituted-2-tetralol expressed by general formula (2): 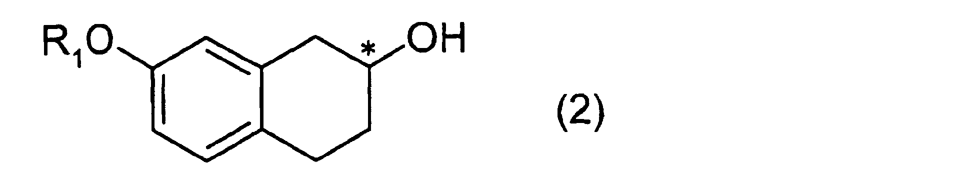 (wherein R₁ represents hydrogen, an alkyl group having 1 to 10 carobn atoms, an aryl group having 6 to 20 carbon atoms, or an aralkyl group having 7 to 20 carbon atoms, and * represents an asymmetric carbon atom), comprising the step of reacting a 7-substituted-2-tetralone expressed by general formula (1): 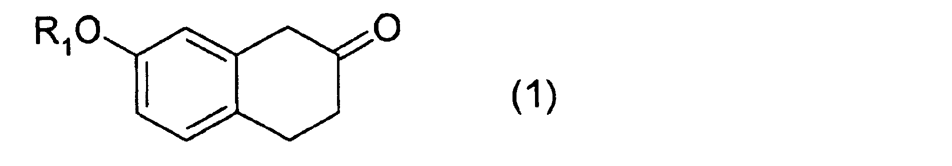 (wherein R₁ represents the same as above) or a bisulfite adduct thereof with a culture broth of microorganism, cells, or a material derived therefrom capable of transforming the 7-substituted-2-tetralone or the bisulfite adduct thereof into the optically active 7-substituted-2-tetralol, wherein the microorganism is a microorganism belonging to a genus selected from the group consisting of *Candida, Debaryomyces,* *Pichia, Kluyveromyces, Metschnikowia, Ogataea, Sporidiobolus, Torulaspora, Geotrichum, Yamadazyma, Endomyces, Dipodascus, Saccharomycopsis, Issatchenkia, Kuraishia, Lipomyces, Lodderomyces, Rhodosporidium, Rhodotorula, Sporobolomyces, Saturnispora, Zygosaccharomyces, Cellulomonas, Jensenia, Arthrobacter, Acidiphilium, Pseudomonas, Rhodococcus, Devosia,* and *Micrococcus.*

2. The process according to Claim 1, wherein the 7-substituted-2-tetralone expressed by formula (1) or the bisulfite adduct thereof is reacted with a culture broth of microorganism, cells, or a material derived therefrom capable of transforming the 7-substituted-2-tetralone or the bisulfite adduct thereof into an optically active 7-substituted-2-tetralol having the (R) configuration to prepare the (R)-7-substituted-2-tetralol, and the microorganism is a microorganism belonging to a genus selected from the group consisting of *Candida, Debaryomyces, Pichia, Kluyveromyces, Metschnikowia, Ogataea, Sporidiobolus, Torulaspora, Geotrichum, Yamadazyma, Arthrobacter, Acidiphilium, Pseudomonas, Rhodococcus,* and *Devosia.*

3. The process according to Claim 1, wherein the 7-substituted-2-tetralone expressed by formula (1) or the bisulfite adduct thereof is reacted with a culture broth of microorganism, cells, or a material derived therefrom capable of transforming the 7-substituted-2-tetralone or the bisulfite adduct thereof into an optically active 7-substituted-2-tetralol having the (S) configuration to prepare the (S)-7-substituted-2-tetralol, and the microorganism is a microorganism belonging to a genus selected from the group consisting of *Candida, Debaryomyces, Endomyces, Dipodascus, Saccharomycopsis, Issatchenkia, Kuraishia, Lipomyces, Lodderomyces, Pichia, Rhodosporidium, Rhodotorula, Sporobolomyces, Sporidiobolus, Saturnispora, Zygosaccharomyces, Cellulomonas, Jensenia, Micrococcus, Rhodococcus,* and *Metschnikowia.*

4. The process according to Claim 1 or 2, wherein the culture broth of microorganism, cells, or a material derived therefrom contains at least one microorganism selected from the group consisting of *Candida magnoliae, Candida maris, Candida catenulate, Candida glabrata, Candida maltosa, Candida albicans, Candida fennica, Debaryomyces hansenii var. hansenii, Pichia anomala, Kluyveromyces polysporus, Metschnikowia bicuspidata var. bicuspidata, Ogataea minuta var. nonfermentans, Sporidiobolus johnsonii, Torulaspora delbrueckii, Geotrichum fermentans, Yamadazyma farinosa, Arthrobacter protophormiae, Acidiphilium cryptum, Pseudomonas putida, Rhodococcus erythropolis*, and *Devosia* *riboflavina*.

5. The process according to Claim 1 or 3, wherein the culture broth of microorganism, cells, or a material derived therefrom contains at least one microorganism selected from the group consisting of *Candida glaebosa, Candida haemulonii, Candida holmii, Candida in termedia, Candida boidinii, Candida pintolopesii, Candida oleophila, Candida sonorensis, Candida tropjcalis, Debaryomyces carsonii, Endomyces decipiens, Dipodascus ovetensis, Saccharomycopsis selenospora, Issatchenkia terricola, Kuraishia* capsulate, *Lipomyces starkeyi, Lodderomyces elongisporus, Metschnikowia gruessii, Pichja wickerhamii, Rhodosporidium toruloides, Rhodotorula araucariae, Sporobolomyces salmonicolor, Sporidiobolus holsaticus, Debaryomyces occidentalis var. occidentalis, Saturnispora dispora, Candida stellata, Zygosaccharomyces bailii, Cellulomonas fimi, Jensenia canicruria, Micrococcus luteus,* and *Rhodococcus erythropolis*.

6. The process according to any one of Claims 1 to 5, wherein R₁ represents a methyl group.

7. A process for preparing an optically active 7-substituted-2-aminotetralin expressed by general formula (5): 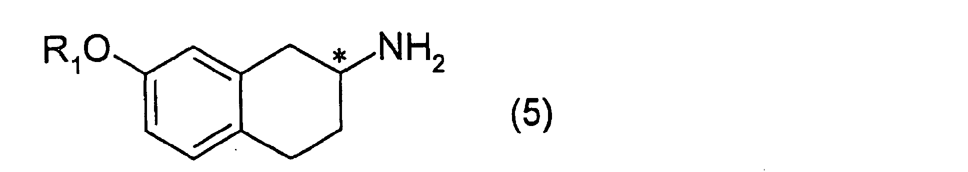 (wherein * represents an asymmetric carbon atom and R₁ represents hydrogen, an alkyl group having 1 to 10 carbon atoms, an aryl group having 6 to 20 carbon atoms, or an aralkyl group having 7 to 20 carbon atoms) or a salt thereof, comprising the steps of: introducing a sulfonyl group to the hydroxy group of an optically active 7-substituted-2-tetralol expressed by general formula (2): 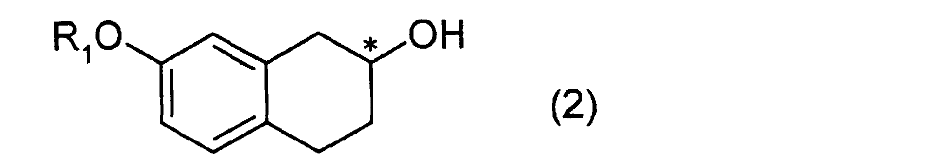 (wherein * and R₁ represents the same as above) to form an optically active 7-substituted-2-sulfonyloxytetralin expressed by general formula (3): 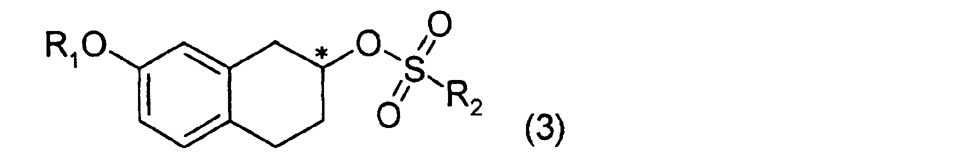 (wherein * and R₁ represent the same as above, and R₂ represents an alkyl group having 1 to 10 carbon atoms, an aryl group having 6 to 20 carbon atoms, an aralkyl group having 7 to 20 carbon atoms, a substituted amino group, or a hydroxy group); introducing a nitrogen substituent using a nitrogen nucleophile to form an optically active 2,7-substituted tetralin expressed by general formula (4): 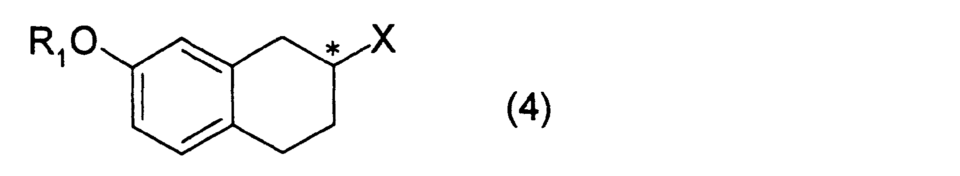 (wherein * and R₁ represents the same as above, and X represents a non-substituted amino group, an alkylamino group having 1 to 10 carbon atoms, an arylamino group having 6 to 20 carbon atoms, an aralkylamino group having 7 to 20 carbon atoms, an amido group having 1 to 20 carbon atoms, an imido group having 2 to 20 carbon atoms, a sulfonylamino group having 1 to 20 carbon atoms, or an azido group) while the configuration is inversed; and, if necessary, transforming the nitrogen substituent to a non-substituted amino group.

8. The process according to Claim 7, wherein an (S)-7-substituted-2-aminotetralin (5) is prepared from an (R)-7-substituted-2-tetralol (2).

9. The process according to Claim 7 or 8, wherein the optically active 7-substituted-2-tetralol (2) is prepared by the process according to any one of Claims 1 to 6.

10. The process according to any one of Claims 7 to 9, wherein the nitrogen nucleophile is ammonia, a metal salt of a phthalimide, or a metal azide, and X in general formula (4) is an amino group, a phthalimido group, or an azido group.

11. The process according to Claim 10, wherein the nitrogen nucleophile is ammonia, and X is an amino group.

12. The process according to any one of Claims 7 to 10, wherein the nitrogen nucleophile is a metal azide, and X is an azido group, and wherein the 2,7-substituted tetralin expressed by general formula (4) is transformed to the optically active 2-substituted-2-aminotetralin expressed by general formula (5) or a salt thereof by reduction.

13. The process according to Claim 12, wherein hydrogen is used in the reduction.

14. An optically active 7-substituted-2-sulfonyloxytetralin expressed by general formula (3): 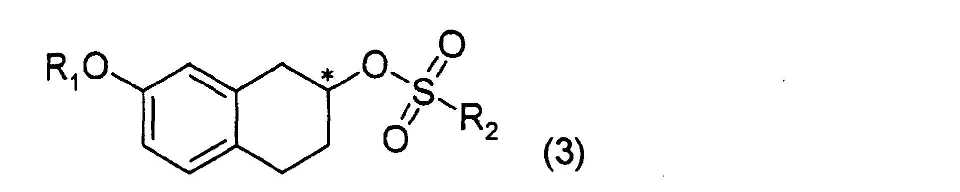 (wherein * represents an asymmetric carbon atom, and R₁ represents hydrogen, an alkyl group having 1 to 10 carbon atoms, an aryl group having 6 to 20 carbon atoms, or an aralkyl group having 7 to 20 carbon atoms, and R₂ represents an alkyl group having 1 to 10 carbon atoms, an aryl group having 6 to 20 carbon atoms, an aralkyl group having 7 to 20 carbon atoms, a substituted amino group, or a hydroxy group).

15. A compound according to Claim 14, wherein R₁ is a methyl group.

16. A compound according to Claim 14 or 15, wherein R₂ is a methyl group, a phenyl group, a p-methylphenyl group, an o-nitrophenyl group, a m-nitrophenyl group, or a p-nitrophenyl group.
